# FASCICULE DE BREVET EUROPEEN

(11) **EP 3 927 400 B1**
(45) Date de publication et mention de la délivrance du brevet: **07.08.2024**
(21) Numéro de dépôt: 20707524.3
(22) Date de dépôt: 18.02.2020
(51) Int. Cl.: A61M 5/315, A61M 5/31

(54) **PROCEDE DE PREPARATION D'UNE SERINGUE**
VERFAHREN ZUR HERSTELLUNG EINER SPRITZE
METHOD FOR PREPARING A SYRINGE

(30) Priorité: 21.02.2019 FR 1901787
(43) Date de publication de la demande: 29.12.2021
(73) Titulaire: Clayens NP, 69740 Genas (FR)
(72) Inventeur: COLLADO, Marc, 69500 Bron (FR); LOPEZ, Marie-Charlotte, 01430 Condamine (FR)
(74) Mandataire: Cabinet Poncet
(86) Numéro de dépôt international: PCT/IB2020/051320
(87) Numéro de publication internationale: WO 2020/170116

(56) Documents cités:
- EP-A1- 2 371 406
- EP-B1- 0 954 439
- WO-A1-2017/220553
- WO-A1-2018/011190
- US-A1- 2007 088 291
- US-A1- 2008 071 228
- US-A1- 2011 186 537
- US-A1- 2012 277 686
- US-A1- 2018 110 933
- US-B2- 9 220 631

## Description

### DOMAINE TECHNIQUE DE L'INVENTION

La présente invention concerne un procédé de préparation d'une seringue, lequel peut être plus particulièrement utilisé pour la fabrication d'une seringue pré-remplie.

De façon connue, une seringue comporte un corps de seringue s'étendant selon une direction longitudinale entre une extrémité proximale par laquelle est destiné à être introduit un piston et une extrémité distale à orifice de sortie. Le corps de seringue comprend un volume intérieur défini par une surface intérieure annulaire (qui est généralement cylindrique). Le piston comprend un joint destiné à venir en contact étanche contre la surface intérieure annulaire, et est introduit dans le corps de seringue par son extrémité proximale. Le déplacement du piston dans le corps de seringue en direction de l'extrémité distale provoque l'éjection du liquide hors du corps de seringue à travers l'orifice de sortie.

La performance d'une seringue est notamment appréciée par les propriétés de glissement du piston à l'intérieur du corps de seringue. On mesure généralement la force de déclenchement et la force de glissement, c'est-à-dire la force qu'il faut appliquer sur le piston pour initier son déplacement, puis la force qu'il faut appliquer sur le piston pour poursuivre sa translation à l'intérieur du corps de seringue.

Ces forces s'avèrent particulièrement critiques pour les seringues pré-remplies à usage médical qui peuvent être disposées sur des appareils médicaux à actionneurs automatisés pour gérer de façon très précise les doses injectées.

Pour une utilisation satisfaisante, une seringue doit présenter une force de déclenchement et une force de glissement les plus faibles possibles pour une injection régulière et précise, et de préférence les plus voisines possibles. De trop grandes forces de déclenchement et de glissement (et éventuellement de trop grands écarts entre ces forces) peuvent provoquer un déplacement saccadé du piston à l'intérieur du corps de seringue, résultant en une injection mal maîtrisée.

Il faut en outre que les propriétés de glissement de la seringue soient durables et répétitibles, car de nombreuses seringues pré-remplies sont stockées plusieurs mois avant d'être utilisées.

Pour obtenir des propriétés de glissement satisfaisantes, il est connu de revêtir la surface intérieure du corps de seringue avec du silicone. Il convient toutefois de limiter autant que possible la quantité de silicone utilisée. En effet, le silicone présente l'inconvénient d'être bio-toxique. Il faut donc limiter les risques d'une migration du silicone dans le liquide destiné à être injecté, risque qui est accru dans le cas des seringues pré-remplies qui peuvent avoir une durée de stockage importante.

Le document US 2012/0277686 A1 décrit un procédé de préparation d'une seringue lors duquel la surface intérieure annulaire du corps de seringue est revêtue de silicone avant d'être soumise à un traitement corona. Ce document mentionne expressément l'importance de l'ordre de ces opérations, un ordre inverse aboutissant à des propriétés de glissement catastrophiques.

Le document US 2008/071228 A1 a également pour enseignement principal un procédé de préparation d'une seringue lors duquel la surface intérieure annulaire du corps de seringue est revêtue de silicone avant d'être soumise à un traitement corona. Dans une variante dudit procédé de préparation, le document US 2008/071228 A1 prévoit en outre l'application d'un traitement corona supplémentaire après l'étape de revêtement par du silicone. Ladite variante consiste ainsi en un procédé de préparation comportant deux étapes de traitement corona (la première avant de revêtir de silicone la surface intérieure annulaire du corps de seringue, et la seconde après avoir revêtu de silicone la surface intérieure annulaire du corps de seringue).

### EXPOSE DE L'INVENTION

Un problème proposé par la présente invention est de proposer une méthode de préparation d'une seringue permettant d'améliorer les propriétés de glissement du piston d'une seringue, notamment par l'obtention d'une force de déclenchement et d'une force de glissement relativement faibles, tout en limitant la quantité de silicone nécessaire.

Simultanément, l'invention vise à obtenir une seringue dont les propriétés de glissement sont durables et répétitibles.

Pour atteindre ces objets ainsi que d'autres, l'invention propose un procédé de préparation d'une seringue, comprenant les étapes successives suivantes :
a) fournir un corps de seringue ayant une surface intérieure annulaire, ledit corps s'étendant autour d'un axe de symétrie longitudinal entre une extrémité distale à orifice de sortie et une extrémité proximale par laquelle est destinée à être introduite une tête de piston comprenant un joint destiné à venir en contact étanche contre la surface intérieure annulaire,
b) appliquer un traitement corona à ladite surface intérieure annulaire,
c) revêtir ladite surface intérieure annulaire d'une couche de silicone de façon à obtenir un corps de seringue à surface intérieure annulaire revêtue de silicone,
d) irradier par rayonnement gamma le corps de seringue ;
selon l'invention, il n'est appliqué qu'une unique étape de traitement corona.

L'invention repose sur l'observation surprenante selon laquelle le fait d'appliquer du silicone sur la surface intérieure annulaire du corps de seringue après que ladite surface intérieure annulaire ait subi un traitement corona, conjugué à une étape ultérieure d'irradiation par rayonnement gamma de la seringue, conduit contre toute attente à des propriétés de glissement particulièrement avantageuses. C'est en tous cas le constat étonnant qu'a pu faire le demandeur, et ce en dépit des enseignements contraires du document US 2012/0277686 A1 et en dépit des enseignements principaux contraires du document US 2008/071228 A1.

L'invention repose plus particulièrement sur l'observation surprenante selon laquelle le fait d'appliquer du silicone sur la surface intérieure annulaire du corps de seringue après que ladite surface intérieure annulaire ait subi un unique traitement corona, conjugué à une étape ultérieure d'irradiation par rayonnement gamma de la seringue, conduit contre toute attente à des propriétés de glissement particulièrement avantageuses. C'est en tous cas le constat étonnant qu'a pu faire le demandeur, et ce en dépit des enseignements secondaires contraires du document US 2008/071228 A1.

L'irradiation par rayonnement gamma du corps de seringue peut en pratique consister en une étape de stérilisation par rayonnement gamma du corps de seringue.

Les essais effectués par le demandeur, en conformité avec la norme ISO 7886-1 - Edition 2017 - Annexe E, ont abouti à une force de déclenchement inférieure à 10 N (et même inférieure à 7,5 N) alors que la surface intérieure annulaire comportait moins de 130 µg de silicone par cm² (et même moins de 80 µg de silicone par cm²).

Avantageusement, lors de l'étape d), le rayonnement gamma peut être appliqué selon une dose comprise entre 10 et 50 KGy.

De préférence, après l'étape b), la surface intérieure annulaire peut présenter une tension de surface supérieure ou égale à 32 dyne/cm, et encore plus préférentiellement comprise entre 35 et 54 dyne/cm. Une telle tension de surface semble permettre de diminuer encore significativement la quantité de silicone nécessaire à l'obtention de propriétés de glissement satisfaisantes.

Avantageusement, lors de l'étape b), le temps de traitement corona peut être compris entre 0,5 et 3 secondes, de préférence entre 0,75 et 1,5 secondes. Un tel temps de traitement corona permet de limiter efficacement la durée du procédé de préparation pour une plus grande productivité. Au-delà de ce temps de traitement, la durée globale du procédé de préparation est allongée sans procurer pour autant une amélioration significative des propriétés de glissement.

De préférence, lors de l'étape b), le traitement corona peut être réalisé avec une intensité de courant électrique comprise entre 0,025 et 0,075 mA, de préférence entre 0,04 et 0,06 mA. Une telle intensité de courant électrique contribue à l'obtention de propriétés de glissement satisfaisantes, tout en limitant les risques de génération d'un arc électrique qui endommagerait sévèrement la surface intérieure annulaire et dégraderait fortement les propriétés de glissement.

Avantageusement, on peut prévoir que, lors de l'étape c), on revêt la surface intérieure annulaire par projection de silicone au moyen d'une buse de pulvérisation déplacée en translation depuis l'extrémité distale vers l'extrémité proximale.

Un tel mode de projection du silicone permet simultanément de limiter et de maîtriser la quantité de silicone nécessaire, tout en favorisant un revêtement homogène sur la surface intérieure annulaire.

De préférence, lors de l'étape c), l'orifice de sortie est obstrué de façon étanche par un bouchon. Cette obstruction permet de limiter des pertes de silicone, et donc de limiter la quantité de silicone nécessaire. Par ailleurs, cela génère des turbulences de flux lors de la pulvérisation du silicone, lesquelles turbulences favorisent une répartition homogène du silicone sur la surface intérieure annulaire.

Avantageusement, lors de l'étape c), la buse de pulvérisation est déplacée selon la direction longitudinale à une vitesse comprise entre 120 et 200 mm/s. La pulvérisation du silicone est ainsi rapide pour ne pas allonger inutilement le procédé de préparation, et limite les risques d'une surcharge en silicone de la surface intérieure annulaire.

Selon une première possibilité, on peut appliquer une unique étape de traitement corona à la surface intérieure annulaire. Une unique étape de traitement corona procure une durée globale relativement restreinte du procédé de préparation,

Avantageusement, le corps de seringue peut avoir une surface intérieure annulaire en copolymère cyclo-oléfine (COC), en polymère cyclo-oléfine (COP), en verre, en polytétrafluoroéthylène (PTFE), en polychlorure de vinyle (PVC), en polypropylène (PP), en polyéthylène (PET), en polycarbonate (PC), ou en polystyrène (PS). Ces matériaux ont tous une tension de surface qui peut être augmentée par effet corona.

De préférence, on peut fournir un joint de piston de seringue en bromobutyle, en chlorobutyle, ou en bromochlorobutyle.

Avantageusement, on peut fournir un joint de piston de seringue présentant, avant introduction dans le corps de seringue, un diamètre extérieur qui est supérieur de 0,27 à 0,47 mm au diamètre intérieur de la surface intérieure annulaire avant son revêtement par le silicone. Un tel jeu négatif entre le joint du piston et la surface intérieure annulaire permet d'obtenir une étanchéité satisfaisante, sans pour autant affecter de façon substantielle les propriétés de glissement.

De préférence, on peut fournir un joint de piston de seringue présentant une dureté comprise entre 50 et 56 Shore A.

Pour faciliter l'introduction et le coulissement du piston dans le corps de seringue, on peut fournir un joint du piston de seringue comportant avantageusement un revêtement de silicone préalablement à son introduction dans le corps de seringue.

De préférence, pour obtenir une seringue pré-remplie, le corps de seringue peut-être rempli d'une substance liquide et assemblé avec une tête de piston comprenant un joint destiné à venir en contact étanche contre la surface intérieure annulaire. Ladite seringue peut avantageusement être soumise à une étape de stérilisation (par exemple par autoclave) pour éliminer d'éventuels germes.

Selon un autre aspect de l'invention, il est proposé un ensemble pour la fabrication d'une seringue, ledit ensemble comportant une tête de piston de seringue, et comportant un corps de seringue ayant une surface intérieure annulaire à laquelle ont été successivement appliquées une unique étape de traitement corona suivie d'une étape de revêtement d'une couche de silicone, de préférence suivie d'une étape d'irradiation par rayonnement gamma.

Avantageusement, après l'étape de traitement corona, la surface intérieure annulaire peut présenter une tension de surface supérieure ou égale à 32 dyne/cm, et de préférence comprise entre 35 et 54 dyne/cm.

De préférence, la surface intérieure annulaire du corps de seringue comporte en moyenne moins de 130 µg de silicone par cm², de préférence moins de 80 µg de silicone par cm².

Selon un autre aspect, l'invention concerne une seringue préparée au moyen d'une tête de piston de seringue et d'un corps de seringue préparé selon le procédé de préparation tel que décrit précédemment.

Avantageusement, dans ladite seringue :
- la surface intérieure annulaire a un diamètre intérieur de 5 mm,
- l'initiation du déplacement de la tête de piston en direction de l'extrémité distale nécessite l'application d'un effort de poussée inférieur à 7,5 N.

Par « silicone », on entend notamment désigner toute substance contenant un ou plusieurs polysiloxanes.

### DESCRIPTION SOMMAIRE DES DESSINS

D'autres objets, caractéristiques et avantages de la présente invention ressortiront de la description suivante de modes de réalisation particuliers, faite en relation avec les figures jointes, parmi lesquelles :
[Fig.1] La figure 1 est une vue schématique en coupe longitudinale d'éléments constitutifs d'une seringue, à savoir un corps de seringue, un bouchon de seringue et une tête de piston de seringue ;
[Fig.2] La figure 2 est un schéma-bloc illustrant un mode de réalisation particulier de procédé selon la présente invention ;
[Fig.3] La figure 3 est un schéma-bloc illustrant un procédé selon des enseignements du document antérieur US 2008/071228 A1 ;
[Fig.4] La figure 4 est une vue schématique en coupe longitudinale d'une étape d'application d'un traitement corona au corps de seringue de la figure 1 ;
[Fig.5] La figure 5 est une vue schématique en coupe longitudinale d'une étape de revêtement de l'intérieur du corps de seringue de la figure 1 par une couche de silicone ; et
[Fig.6] La figure 6 est une vue schématique en coupe longitudinale d'une seringue pré-remplie.

### DESCRIPTION DES MODES DE REALISATION PREFERES

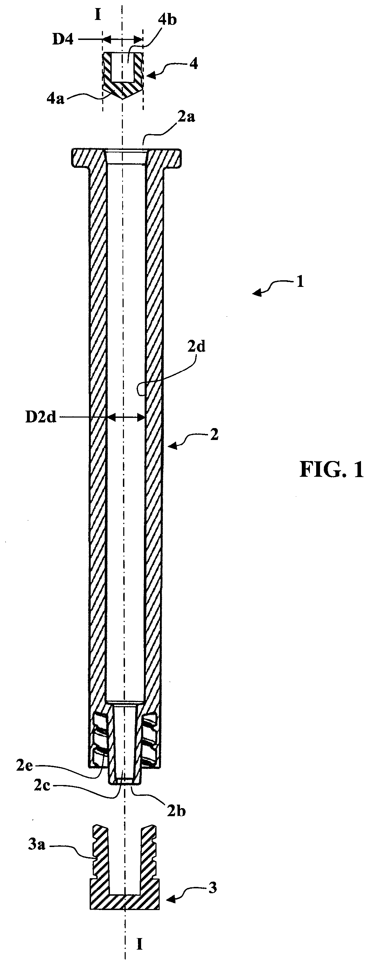

Sur la figure 1 sont illustrés au moyen d'une vue schématique en coupe longitudinale plusieurs éléments constitutifs d'une seringue 1, à savoir un corps 2 de seringue, un bouchon 3 de seringue et une tête de piston 4 de seringue.

Le corps 2 de seringue s'étend, autour d'un axe de symétrie longitudinal I-I, entre une extrémité proximale 2a, par laquelle est destinée à être introduite la tête de piston 4, et une extrémité distale 2b à orifice 2c de sortie. Le corps 2 de seringue comprend un volume intérieur défini par une surface intérieure annulaire 2d (qui est généralement cylindrique).

La tête de piston 4 forme ici un joint 4a destiné à venir en contact étanche et glissant contre la surface intérieure annulaire 2d, et est introduite dans le corps 2 de seringue par son extrémité proximale 2a. La tête de piston 4 comporte une interface de connexion 4b à une tige de piston, laquelle interface de connexion 4b peut présenter diverses configurations.

Le bouchon 3 de seringue est destiné à venir obstruer de façon étanche l'orifice 2c de sortie. En l'espèce, le bouchon 3 est destiné à coopérer par vissage au moyen d'un filetage mâle 3a avec l'extrémité distale 2b qui est quant à elle pourvue d'un filetage femelle 2e.

La surface intérieure annulaire 2d du corps 2 peut être en copolymère cyclo-oléfine (COC), en polymère cyclo-oléfine (COP), en verre, en polytétrafluoroéthylène (PTFE), en polychlorure de vinyle (PVC), en polypropylène (PP), en polyéthylène (PET), en polycarbonate (PC), ou en polystyrène (PS).

De son côté, le joint 4a du piston de seringue peut être en bromobutyle, en chlorobutyle, ou en bromochlorobutyle.

Pour assurer une étanchéité satisfaisante, le joint 4a du piston de seringue présente avantageusement un diamètre extérieur D4 qui, avant son introduction dans le corps 2 de seringue, est supérieur de 0,27 mm à 0,47 mm au diamètre intérieur D2d de la surface intérieure annulaire 2d (avant son revêtement par du silicone comme le prévoit le procédé de la présente invention).

Le joint 4a du piston de seringue présente de préférence une dureté comprise entre 50 et 56 Shore A.

Pour faciliter son introduction dans le corps 2 de seringue, mais également son glissement ultérieur dans ledit corps 2, le joint 4a du piston de seringue peut comporter un revêtement de silicone sur sa surface extérieure.

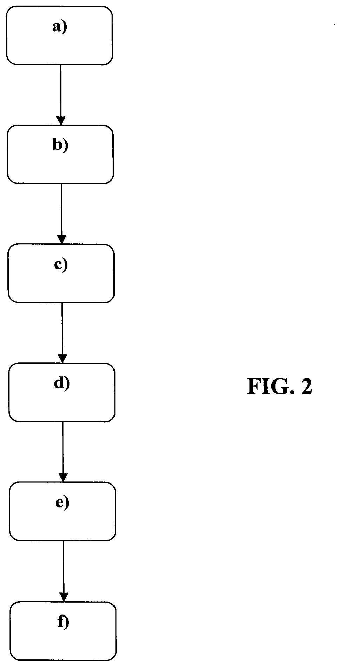

Sur la figure 2 est représenté un schéma-bloc illustrant un mode de réalisation de procédé selon la présente invention. Ledit procédé comprend les étapes successives suivantes :
a) fournir un corps 2 de seringue (tel que celui de la figure 1), ayant une surface intérieure annulaire 2d, ledit corps 2 s'étendant autour d'un axe de symétrie longitudinal 1-1 entre une extrémité distale 2b à orifice de sortie 2c et une extrémité proximale 2a par laquelle est destiné à être introduit une tête de piston 4 comprenant un joint 4a destiné à venir en contact étanche contre la surface intérieure annulaire 2d,
b) appliquer un traitement corona à ladite surface intérieure annulaire 2d,
c) revêtir ladite surface intérieure annulaire 2d d'une couche de silicone de façon à obtenir un corps 2 de seringue à surface intérieure annulaire 2d revêtue de silicone,
d) irradier par rayonnement gamma le corps 2 de seringue,
selon l'invention, il n'est appliqué qu'une unique étape de traitement corona (l'étape b)).

L'irradiation par rayonnement gamma du corps de seringue peut en pratique consister en une étape de stérilisation par rayonnement gamma du corps de seringue.

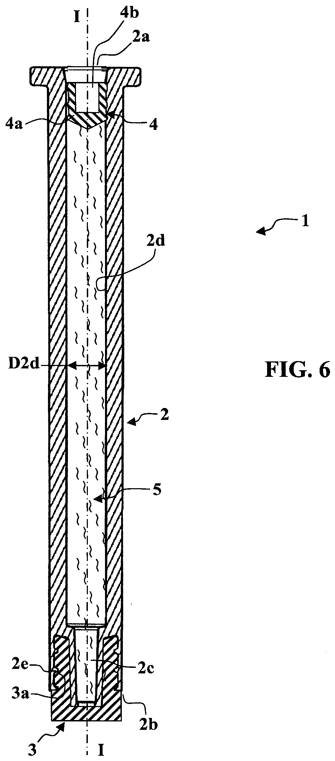

Pour obtenir une seringue 1 pré-remplie telle qu'illustrée sur la figure 6, lors d'une étape e), le corps 2 de seringue peut être rempli d'une substance liquide 5 destinée à être injectée à un patient, et assemblé avec une tête de piston 4 comprenant un joint 4a destiné à venir en contact étanche contre la surface intérieure annulaire 2d. La seringue 1 pré-remplie est de préférence soumise ensuite à une étape f) de stérilisation, par exemple par autoclave, pour éliminer d'éventuels germes.

Il est à noter que, en alternative, le remplissage du corps 2 de seringue par la substance liquide 5 destinée à être injectée à un patient peut être effectué avant l'étape d). La seringue 1 pré-remplie peut alors ne subir qu'une seule stérilisation, à savoir l'irradiation par rayonnement gamma de l'étape d).

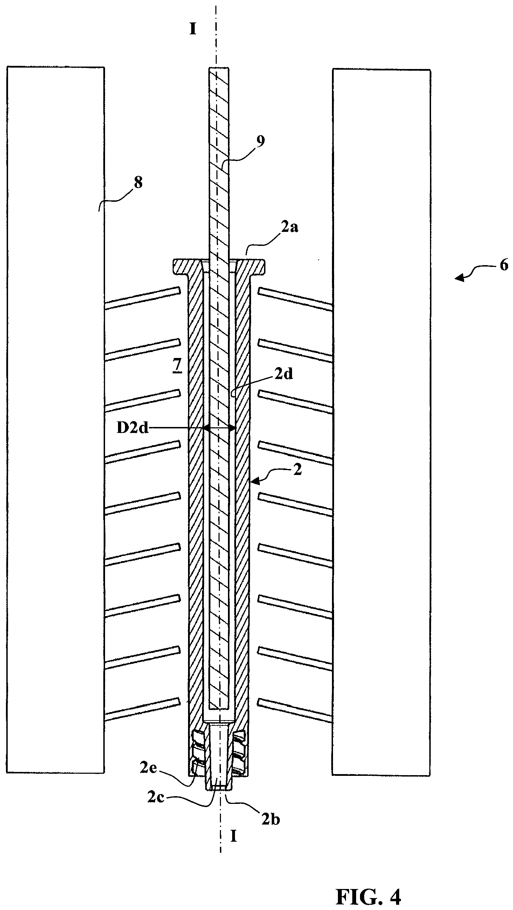

L'étape b) est schématiquement illustrée sur la figure 4. Sur cette figure, on voit que le corps 2 est introduit dans un dispositif 6 de traitement corona.

Plus spécifiquement, le corps 2 est introduit dans un logement 7 défini par un matériau diélectrique 8 (jouant le rôle de contre-électrode ou de masse). Une électrode 9 filaire conductrice de courant est ensuite introduite dans le corps 2 de seringue selon l'axe de symétrie longitudinal I-I, en correspondance de toute la longueur de la surface intérieure annulaire 2d. Un générateur à haute fréquence et haute tension alternative est connecté entre l'électrode 9 et le matériau diélectrique 8, et génère ainsi des décharges électriques 8 entre l'électrode 9 et le matériau diélectrique à travers le corps 2 de seringue, ce qui a pour effet de modifier la tension de surface de la surface intérieure annulaire 2d.

Le temps de traitement corona est compris entre 0,5 et 3 secondes, de préférence entre 0,75 et 1,5 secondes. L'intensité de courant électrique délivrée par le générateur est comprise entre 0,025 et 0,075 mA, de préférence entre 0,04 et 0,06 mA.

Des seringues à propriétés de glissement remarquables ont été obtenues avec un traitement corona effectué jusqu'à obtenir sur la surface intérieure annulaire 2d une tension de surface supérieure ou égale à 32 dyne/cm, et de préférence comprise entre 35 et 54 dyne/cm.

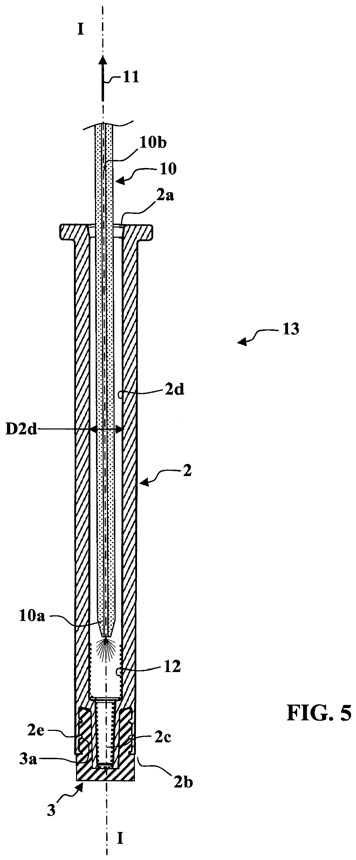

L'étape c) est schématiquement illustrée sur la figure 5. Sur cette figure, on voit que le corps 2 est traité par un dispositif 13 de siliconage.

Plus spécifiquement, on introduit dans le corps 2 une buse de pulvérisation 10 pour injecter du silicone afin de revêtir la surface intérieure annulaire 2d d'une couche 12 de silicone (illustrée schématiquement en trait discontinus).

En pratique, la buse de pulvérisation 10 est engagée dans le corps 2 selon l'axe longitudinal I-I de façon que son extrémité libre 10a se trouve à proximité de l'extrémité distale 2b du corps 2. On commence alors l'injection de silicone dans le conduit 10b de la buse de pulvérisation 10. Simultanément, on déplace la buse de pulvérisation 10 selon un mouvement de translation selon la direction longitudinale 1-1 (illustré par la flèche 11) depuis l'extrémité distale 2b vers l'extrémité proximale 2a, de préférence selon une vitesse constante comprise entre 120 et 200 mm/s.

Sur la figure 5, l'orifice de sortie 2c est obstrué de façon étanche par le bouchon 3. Cette obstruction permet de limiter des pertes de silicone, et donc de limiter la quantité de silicone nécessaire. Mais surtout, il s'est avéré que cette obstruction génère des turbulences de flux lors de la pulvérisation du silicone, lesquelles turbulences favorisent une répartition homogène du silicone sur la surface intérieure annulaire 2d.

Des seringues à propriétés de glissement remarquables ont été obtenues avec un siliconage menant à une surface intérieure annulaire 2d comportant en moyenne moins de 130 µg de silicone par cm², de préférence moins de 80 µg de silicone par cm².

A l'issue de l'étape c), on peut obtenir un ensemble comportant une tête de piston 4 de seringue et un corps 2 de seringue ayant une surface intérieure annulaire 2d à laquelle ont été appliquées successivement une unique étape de traitement corona suivie d'une étape de revêtement d'une couche de silicone. Cet ensemble peut être vendu en tant que tel à un laboratoire, avant ou après la réalisation de l'étape d) d'irradiation par rayonnement gamma.

Selon une première possibilité, le corps dudit ensemble a subi, avant livraison au laboratoire, l'étape d) d'irradiation par rayonnement gamma. Le laboratoire procède alors au remplissage du corps 2 de seringue par la substance liquide 5 destinée à être injectée à un patient et à l'assemblage du corps 2 de seringue avec la tête de piston 4, puis soumet la seringue 1 pré-remplie ainsi formée à une étape de stérilisation (par exemple par autoclave).

Selon une deuxième possibilité, le corps dudit ensemble n'a pas subi, avant livraison au laboratoire, l'étape d) d'irradiation par rayonnement gamma. Le laboratoire procède alors au remplissage du corps 2 de seringue par la substance liquide 5 destinée à être injectée à un patient et à l'assemblage du corps 2 de seringue avec la tête de piston 4, puis effectue lui-même l'étape d) d'irradiation en soumettant la seringue 1 pré-remplie ainsi formée à un rayonnement gamma. L'étape d) peut alors constituer une étape de stérilisation.

De préférence, le rayonnement gamma est appliqué selon une dose comprise entre 10 et 50 KGy.

Comme on le constate, lors du procédé illustré sur la figure 2, il n'est appliqué qu'une unique étape de traitement corona, à savoir l'étape b).

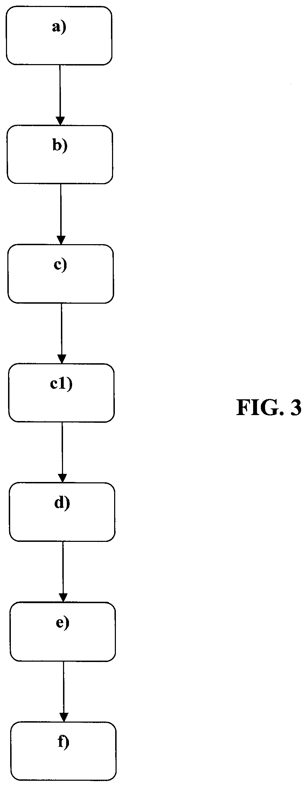

Sur la figure 3 est illustré un schéma-bloc illustrant un procédé selon les enseignements du document US 2008/071228 A1. Le procédé de la figure 3 diffère du procédé de la présente invention illustré sur la figure 2 en ce qu'il comprend deux étapes b) et c1) de traitement corona : la première avant de revêtir de silicone la surface intérieure annulaire du corps de seringue, et la seconde après avoir revêtu de silicone la surface intérieure annulaire du corps de seringue.

De façon plus précise, dans le procédé de la figure 3, entre les étapes c) et d), on effectue une étape c1) de traitement corona à la surface intérieure annulaire 2d. Les autres étapes a), b), c), d), e) et f) sont inchangées.

### Test N°1

Les tableaux 1A à 1F ci-dessous récapitulent des résultats d'un test N°1 de glissement réalisé sur des seringues 1 pré-remplies obtenues par le procédé selon l'invention illustré sur le schéma-bloc de la figure 2.

Les seringues 1 testées présentaient un corps 2 de seringue en COC fabriqué par injection, ayant un diamètre nominal intérieur D2d de 5 mm et une capacité nominale de 1 ml. Le joint 4a du piston de seringue était en bromobutyle présentant une dureté comprise entre 50 et 56 Shore A, et comportait un diamètre extérieur de 5,37 mm (± 0,1).

Le COC utilisé était du TOPAS^{®} commercialisé par la société TOPAS Advanced Polymers GmbH.

Pour le siliconage de la surface intérieure annulaire 2d, il a été utilisé du silicone commercialisé par la société NuSil Technology LLC, déposée à l'aide d'une buse de pulvérisation 10 déplacée en translation à une vitesse comprise entre 120 et 200 mm/s.

Le temps de traitement corona était de 0,75 à 1,5 secondes, avec une intensité de courant électrique comprise entre 0,040 et 0,060 mA.

Avec le procédé de fabrication de la figure 2, la surface intérieure annulaire 2d présentait :
- à l'issue de l'étape b) de traitement corona, une tension de surface comprise entre 35 et 54 dyne/cm,
- à l'issue de l'étape c) de siliconage, en moyenne 750 à 850 µg de silicone répartis sur une surface 12 cm².

Les tests ont été effectués selon la norme ISO 7886-1 - Edition 2017 - Annexe E.

Lors dudit test, on a procédé à des mesures des forces exercées sur le piston (joint 4a), lequel était animé d'une vitesse de déplacement de 100 mm/min. Fs représente la force requise pour amorcer le mouvement du piston, en N. F min représente la force minimale mesurée pendant le déplacement du piston, en N.

F représente la force moyenne mesurée pendant le déplacement du piston, en N.

F max représente la force maximale pendant le déplacement du piston, en N. Des mesures ont été effectuées sur un maximum de 10 échantillons, tous les mois pendant 6 mois.

**[Tableau 1A]**

| Poussée effectuée après une durée de stockage de 1 mois | | | | | | | | | | | |
|---|---|---|---|---|---|---|---|---|---|---|---|
| | Numéros d'échantillon | | | | | | | | | | |
| | 1 | 2 | 3 | 4 | 5 | 6 | 7 | 8 | 9 | 10 | Moyenne |
| Fs (N) | 5.46 | 2.36 | 2.36 | * | 2.57 | 2.79 | 3.36 | ** | 2.57 | 2.18 | 2.96 |
| F min (N) | 0.83 | 0.78 | 0.8 | * | 0.78 | 0.8 | 0.84 | ** | 0.81 | 0.86 | 0.81 |
| F (N) | 0.92 | 0.88 | 0.89 : | * | 0.87 | 0.93 | 0.91 | ** | 0.89 | 0.95 | 0.91 |
| F max (N) | 1.07 | 1.02 | 1.02 | * | 0.99 | 1.11 | 1.05 | ** | 1.05 | 1.13 | 1.06 |

| | | | | | | | | | | | |
|---|---|---|---|---|---|---|---|---|---|---|---|
| * erreur de manipulation par l'opérateur lors du test ** erreur due à l'absence de revêtement de silicone sur la surface intérieure annulaire | | | | | | | | | | | |

**[Tableau 1B]**

| Poussée effectuée après une durée de stockage de 2 mois | | | | | | | | | | | |
|---|---|---|---|---|---|---|---|---|---|---|---|
| | Numéros d'échantillon | | | | | | | | | | |
| | 1 | 2 | 3 | 4 | 5 | 6 | 7 | 8 | 9 | 10 | Moyenne |
| Fs (N) | 2.58 | 2.6 | 2.43 | 2.18 | 5.98 | 1.98 | 3.84 | 2.21 | 2.58 | 5.83 | 3.22 |
| F min (N) | 0.77 | 0.74 | 0.74 | 0.71 | 0.5 | 0.71 | 0.81 | 0.75 | 0.69 | 0.74 | 0.72 |
| F (N) | 0.84 | 0.81 | 0.83 | 0.78 | 0.87 | 0.78 | 0.86 | 0.83 | 0.79 | 0.83 | 0.82 |
| F max (N) | 0.95 | 0.9 | 0.96 | 0.89 | 1,01 | 0.9 | 0.99 | 0.96 | 0.89 | 0.96 | 0.94 |

**[Tableau 1C]**

| Poussée effectuée après une durée de stockage de 3 mois | | | | | | | | | | | |
|---|---|---|---|---|---|---|---|---|---|---|---|
| | Numéros d'échantillon | | | | | | | | | | |
| | 1 | 2 | 3 | 4 | 5 | 6 | 7 | 8 | 9 | 10 | Moyenne |
| Fs (N) | 2.25 | 5.15 | 1.13 | 1.91 | 2.12 : | 2.46 | 1.88 | 2 | 2.88 | 5.79 | 2.76 |
| F min (N) | 0.74 | 0.71 | 0.75 | 0.69 | 0.71 : | 0.66 | 0.71 | 0.65 | 0.66 | 0.56 | 0.68 |
| F (N) | 0.83 | 0.78 | 0.85 | 0.78 | 0.78 : | 0.74 | 0.78 | 0.76 | 0.74 | 0.86 | 0.79 |
| F max (N) | 0.95 | 0.92 | 0.98 | 0.89 | 0.9 | 0.86 | 0.92 | 0.89 | 0.83 | 0.98 | 0.90 |

**[Tableau 1D]**

| Poussée effectuée après une durée de stockage de 4 mois | | | | | | | | | | | |
|---|---|---|---|---|---|---|---|---|---|---|---|
| | Numéros d'échantillon | | | | | | | | | | |
| | 1 | 2 | 3 | 4 | 5 | 6 | 7 | 8 | 9 | 10 | Moyenne |
| Fs (N) | 2.71 | 5.36 | 3.41 | 3.51 | 2.55 | 2.57 | 3 | 3.15 | 5.24 | ** | 3.50 |
| F min (N) | 0.71 | 0.68 | 0.72 | 0.77 | 0.65 | 0;54 | 0.74 | 0.71 | 0.72 | ** | 0.71 |
| F (N) | 0.79 | 0.77 | 0.84 | 0.85 | 0.76 | 0.81 | 0.83 | 0.82 | 0.81 | ** | 0.81 |
| F max (N) | 0.9 | 0.92 | 1.01 | 1.01 | 0.9 | 0.92 | 0.98 | 0.98 | 0.95 | ** | 0.95 |

| | | | | | | | | | | | |
|---|---|---|---|---|---|---|---|---|---|---|---|
| ** erreur due à l'absence de revêtement de silicone sur la surface intérieure annulaire | | | | | | | | | | | |

**[Tableau 1E]**

| Poussée effectuée après une durée de stockage de 5 mois | | | | | | | | | | | |
|---|---|---|---|---|---|---|---|---|---|---|---|
| | Numéros d'échantillon | | | | | | | | | | |
| | 1 | 2 | 3 | 4 | 5 | 6 | 7 | 8 | 9 | 10 | Moyenne |
| Fs (N) | 3.38 | 6.89 | 1.97 | 2.22 | 2.25 | 1.64 | 2.4 | 2.69 | 1.8 | 6.55 | 3.18 |
| F min (N) | 0.74 | 0.75 | 0.78 | 0.63 | 0.68 | 0.72 | 0.68 | 0.62 | 0.68 | 0.71 | 0.70 |
| F (N) | 0.84 | 0.87 | 0.89 | 0.74 | 0.75 | 0.83 | 0.78 | 0.78 | 0.76 | 0.81 | 0.81 |
| F max (N) | 1.01 | 1,01 | 1.07 | 0.89 | 0.87 | 0.98 | 0.96 | 0.89 | 0.89 | 0.96 | 0.95 |

**[Tableau 1F]**

| Poussée effectuée après une durée de stockage de 6 mois | | | | | | | | | | | |
|---|---|---|---|---|---|---|---|---|---|---|---|
| | Numéros d'échantillon | | | | | | | | | | |
| | 1 | 2 | 3 | 4 | 5 | 6 | 7 | 8 | 9 | 10 | Moyenne |
| Fs (N) | 2.66 | 2.12 | 2.49 | 2.27 | 2.72 | 2.39 | 1.25 | 2.54 | 3.15 | 7.09 | 2.87 |
| F min (N) | 0.72 | 0.71 | 0.65 | 0.75 | 0.63 | 0.65 | 0.62 | 0.57 | 0.71 | 0.78 | 0.68 |
| F (N) | 0.77 | 0.77 | 0.74 | 0.85 | 0.71 | 0.69 | 0.69 | 0.65 | 0.78 | 0.88 | 0.75 |
| F max (N) | 0.9 | 0.87 | 0.89 | 0.96 | 0.84 | 0.81 | 0.83 | 0.78 | 0.92 | 1.05 | 0.89 |

Un bilan du test N°1 peut être synthétisé par le tableau suivant.

**[Tableau 1G]**

| | Durées de stockage | | | | | | |
|---|---|---|---|---|---|---|---|
| | 1 mois | 2 mois | 3 mois | 4 mois | 5 mois | 6 mois | Moyenne |
| Fs (N) | 2.96 | 3.22 | 2.76 | 3.50 | 3.18 | 2.87 | 3.08 |
| F (N) | 0.91 | 0.82 | 0.79 | 0.81 | 0.81 | 0.75 | 0.82 |
| Fs / F | 3.27 | 3.92 | 3.49 | 4.33 | 3.95 | 3.81 | 3.79 |

Selon une première observation, la moyenne de la force de déclenchement Fs est de 3,08 N, tandis que la force moyenne de glissement F est en moyenne de 0,82 N.

Selon une autre observation, pour une surface intérieure annulaire 2d ayant un diamètre intérieur D2d de 5 mm, l'initiation du déplacement du piston 4 en direction de l'extrémité distale nécessite l'application d'un effort de poussée inférieur à 7,5 N, et en moyenne inférieure à 3,5 N.

Selon une dernière observation, le rapport de la force de déclenchement Fs sur la force moyenne de glissement F est en moyenne de 3,79, de sorte que la force de déclenchement Fs est relativement proche de la force de glissement moyenne F, ce qui limite le risque de déplacement saccadé de la tête de piston 4.

### Test N°2

A titre de comparaison, il a été effectué un test N°2 pour des seringues 1 fabriquées selon le même procédé de la figure 2, dans les mêmes conditions et avec les mêmes matériaux que les seringues 1 objets du test récapitulé par les tableaux 1A à 1F, mais en inversant l'ordre des étapes b) (traitement corona) et c) (siliconage). En d'autres termes, le test N°2 concerne le procédé faisant l'objet de l'enseignement principal du document US 2008/071228 A1.

Des mesures ont été effectuées sur un maximum de 5 échantillons tous les mois pendant 6 mois.

Les résultats sont récapitulés dans les tableaux 2A à 2F ci-dessous.

**[Tableau 2A]**

| Poussée effectuée après une durée de stockage de 1 mois | | | | | | |
|---|---|---|---|---|---|---|
| | Numéros d'échantillon | | | | | |
| | 1 | 2 | 3 | 4 | 5 | Moyenne |
| Fs (N) | 13.09 | 13.39 | 12.55 | 13.13 | 13.37 | 13.11 |
| F min (N) | 1,31 | 1.28 | 1.3 | 1.24 | 1.33 | 1.29 |
| F (N) | 2.4 | 2.34 | 2.44 | 2.27 | 2.16 | 2.32 |
| F max (N) | 2.68 | 2.59 | 2.73 | 2.65 | 2.65 | 2.66 |

**[Tableau 2B]**

| Poussée effectuée après une durée de stockage de 2 mois | | | | | | |
|---|---|---|---|---|---|---|
| | Numéros d'échantillon | | | | | |
| | 1 | 2 | 3 | 4 | 5 | Moyenne |
| Fs (N) | 16.92 | 13.95 | 13.04 | 13.88 | 14.09 | 14.38 |
| F min (N) | 0 | 1.3 | 1.42 | 1.3 | 1.24 | 1.05 |
| F (N) | 2.49 | 2.23 | 2.2 | 3.69 | 2.2 | 2.56 |
| F max (N) | 2.82 | 2.41 | 2.46 | 4.49 | 2.43 | 2.92 |

**[Tableau 2C]**

| Poussée effectuée après une durée de stockage de 3 mois | | | | | | |
|---|---|---|---|---|---|---|
| | Numéros d'échantillon | | | | | |
| | 1 | 2 | 3 | 4 | 5 | Moyenne |
| Fs (N) | 14.68 | 15.08 | 13.4 | | | 14.39 |
| F min (N) | 1.28 | 1.27 | 1.24 | | | 1.26 |
| F (N) | 2.41 | 2.14 | 2.06 | | | 2.20 |
| F max (N) | 2.71 | 2.28 | 2.18 | | | 2.39 |

**[Tableau 2D]**

| Poussée effectuée après une durée de stockage de 4 mois | | | | | | |
|---|---|---|---|---|---|---|
| | Numéros d'échantillon | | | | | |
| | 1 | 2 | 3 | 4 | 5 | Moyenne |
| Fs (N) | 14.5 | 14.4 | 16.32 | 14.46 | 14.61 | 14.86 |
| F min (N) | 1.25 | 1.34 | 0.89 | 0.01 | 1.68 | 1.03 |
| F (N) | 2.22 | 1.97 | 2.3 | 2.21 | 3.52 | 2.44 |
| F max (N) | 2.44 | 2.1 | 2.49 | 2.38 | 5.25 | 2.93 |

**[Tableau 2E]**

| Poussée effectuée après une durée de stockage de 5 mois | | | | | | |
|---|---|---|---|---|---|---|
| | Numéros d'échantillon | | | | | |
| | 1 | 2 | 3 | 4 | 1 5 | Moyenne |
| Fs (N) | * | * | * | | | |
| F min (N) | * | * | * | | | |
| F (N) | * | * | * | | | |
| F max (N) | * | * | * | | | |

| | | | | | | |
|---|---|---|---|---|---|---|
| * erreur de manipulation par l'opérateur lors du test | | | | | | |

**[Tableau 2F]**

| Poussée effectuée après une durée de stockage de 6 mois | | | | | | |
|---|---|---|---|---|---|---|
| | Numéros d'échantillon | | | | | |
| | 1 | 2 | 3 | 1 4 | 5 | Moyenne |
| Fs (N) | 14.98 | 13.46 | 15.8 | 15.46 | | 14.93 |
| F min (N) | 1.59 | 1.25 | 1.82 | 1.33 | | 1.50 |
| F (N) | 2.33 | 2.84 | 3.56 | 2.27 | | 2.75 |
| F max (N) | 2.47 | 3.19 | 4.44 | 2.55 | | 3.16 |

Un bilan du test N°2 peut être synthétisé par le tableau suivant.

**[Tableau 2G]**

| | Durées de stockage | | | | | | |
|---|---|---|---|---|---|---|---|
| | 1 mois | 2 mois | 3 mois | 4 mois | 5 mois | 6 mois | Moyenne |
| Fs (N) | 13.11 | 14.38 | 14.39 | 14.86 | * | 14.93 | 14.33 |
| F (N) | 2.32 | 2.56 | 2.20 | 2.44 | * | 2.75 | 2.45 |
| Fs / F | 5.65 | 5.62 | 6.54 | 6.09 | * | 5.43 | 5.87 |

| | | | | | | | |
|---|---|---|---|---|---|---|---|
| * erreur de manipulation par l'opérateur lors du test | | | | | | | |

Selon une première observation, la moyenne de la force de déclenchement Fs est de 14,33 N, tandis que la force moyenne de glissement F est en moyenne de 2,45 N.

Selon une autre observation, pour une surface intérieure annulaire 2d ayant un diamètre intérieur D2d de 5 mm, l'initiation du déplacement de la tête de piston 4 en direction de l'extrémité distale nécessite l'application d'un effort de poussée en moyenne de 14,93 N, ce qui est très nettement supérieur à celui obtenu dans le cadre du test N°1 (en moyenne inférieur à 3,5 N).

Selon une dernière observation, le rapport de la force de déclenchement Fs sur la force moyenne de glissement F est en moyenne de 5,87, ce qui est nettement supérieur à la valeur obtenue dans le cadre du test N°1 (3,79).

### Test N°3

Toujours à titre de comparaison, il a été effectué un test N°3 pour des seringues 1 fabriquées selon le procédé de la figure 3, dans les mêmes conditions et avec les mêmes matériaux que les seringues 1 objets du test N°1 récapitulés par les tableaux 1A à 1F. Le procédé de fabrication des seringues 1 de ce test N°3 diffère ainsi du procédé de fabrication des seringues 1 du test N°1 par l'ajout d'une étape c1) de traitement corona de ladite surface intérieure annulaire 2d après l'étape c) (siliconage). En d'autres termes, le test N°3 concerne la variante de procédé enseignée par le document antérieur US 2008/071228 A1.

Des mesures ont été effectuées sur un maximum de 5 échantillons tous les mois pendant 6 mois.

Les résultats sont récapitulés dans les tableaux 3A à 3F ci-dessous.

**[Tableau 3A]**

| Poussée effectuée après une durée de stockage de 1 mois | | | | | | |
|---|---|---|---|---|---|---|
| | Numéros d'échantillon | | | | | |
| | 1 | | 3 | 4 | 5 | Moyenne |
| Fs (N) | 9.82 | 9.64 | 10.91 | 10.13 | | 10.13 |
| F min (N) | 1.19 | 1.24 | 1.21 | 1.21 | | 1.21 |
| F (N) | 1.92 | 1.89 | 2.01 | 1.94 | | 1.94 |
| F max (N) | 2.06 | 1.97 | 2.09 | 2.04 | | 2.04 |

**[Tableau 3B]**

| Poussée effectuée après une durée de stockage de 2 mois | | | | | | |
|---|---|---|---|---|---|---|
| | Numéros d'échantillon | | | | | |
| | 1 | 2 | 3 | 4 | 5 | Moyenne |
| Fs (N) | 9.55 | 9.55 | 9.27 | 9.57 | 9.67 | 9.52 |
| F min (N) | 1.13 | 1,21 | 1.25 | 1.33 | 1.18 | 1.22 |
| F (N) | 1.79 | 1.93 | 1.81 | 1.9 | 1.83 | 1.85 |
| F max (N) | 1.85 | 2.04 | 1.89 | 2.04 | 1.97 | 1.96 |

**[Tableau 3C]**

| Poussée effectuée après une durée de stockage de 3 mois | | | | | | |
|---|---|---|---|---|---|---|
| | Numéros d'échantillon | | | | | |
| | 1 | 2 | 3 | 4 | 5 | Moyenne |
| Fs (N) | 10.97 | 13.3 | 11.21 | | | 11.83 |
| F min (N) | 1.07 | 1.31 | 1.25 | | | 1.21 |
| F (N) | 1.95 | 2.06 | 2 | | | 2.00 |
| F max (N) | 2.09 | 2.15 | 2.12 | | | 2.12 |

**[Tableau 3D]**

| Poussée effectuée après une durée de stockage de 4 mois | | | | | | |
|---|---|---|---|---|---|---|
| | Numéros d'échantillon | | | | | |
| | 1 | 2 | 3 | 4 | 5 | Moyenne |
| Fs (N) | 11.02 | 11 | 11.27 | 11.94 | 9.99 | 11.04 |
| F min (N) | 1.16 | 1.09 | 0.43 | 1.25 | 0.98 | 0.98 |
| F (N) | 1.83 | 1.72 | 1.72 | 1.87 | 1.72 | 1.77 |
| F max (N) | 1.98 | 1.82 | 1.8 | 1.97 | 1.82 | 1.88 |

**[Tableau 3E]**

| Poussée effectuée après une durée de stockage de 5 mois | | | | | | |
|---|---|---|---|---|---|---|
| | Numéros d'échantillon | | | | | |
| | 1 | 2 | 3 | 4 | 5 | Moyenne |
| Fs (N) | 9.72 | 9.82 | 11.66 | | | 10.40 |
| F min (N) | 1.06 | 1.12 | 0.37 | | | 0.85 |
| F (N) | 1.69 | 1.74 | 1.93 | | | 1.79 |
| F max (N) | 1.79 | 1.82 | 2.04 | | | 1.88 |

**[Tableau 3F]**

| Poussée effectuée après une durée de stockage de 6 mois | | | | | | |
|---|---|---|---|---|---|---|
| | Numéros d'échantillon | | | | | |
| | 1 | 2 | 3 | 4 | 5 | Moyenne |
| Fs (N) | 11.1 | * | * | * | * | 11.1 |
| F min (N) | 0.79 | 1.13 | 1 | 0.94 | 1.18 | 1.01 |
| F (N) | 1.79 | 1.81 | 1.67 | 1.79 | 1.8 | 1.77 |
| F max (N) | 1.89 | 1.97 | 1.8 | 1.92 | 1.89 | 1.89 |

| | | | | | | |
|---|---|---|---|---|---|---|
| * erreur de manipulation par l'opérateur lors du test | | | | | | |

Un bilan du test N°3 peut être synthétisé par le tableau suivant.

**[Tableau 3G]**

| | Durées de stockage | | | | | | |
|---|---|---|---|---|---|---|---|
| | 1 mois | 2 mois | 3 mois | 4 mois | 5 mois | 6 mois | Moyenne |
| Fs (N) | 10.13 | 9.52 | 11.83 | 11.04 | 10.40 | 11.1 | 10.67 |
| F (N) | 1.94 | 1.85 | 2.00 | 1.77 | 1.79 | 1.77 | 1.85 |
| Fs / F | 5.22 | 5.15 | 5.92 | 6.24 | 5.81 | 6.27 | 5.77 |

Selon une première observation, la moyenne de la force de déclenchement Fs est de 10,67 N, tandis que la force moyenne de glissement F est en moyenne de 1,85 N.

Selon une autre observation, pour une surface intérieure annulaire 2d ayant un diamètre intérieur D2d de 5 mm, l'initiation du déplacement de la tête de piston 4 en direction de l'extrémité distale nécessite l'application d'un effort de poussée en moyenne de 11,83 N, ce qui est très nettement supérieur à celui obtenu dans le cadre du test N°1 (en moyenne inférieur à 3,5 N).

Selon une dernière observation, le rapport de la force de déclenchement Fs sur la force moyenne de glissement F est en moyenne de 5,77, ce qui est légèrement inférieur à la valeur obtenue dans le cadre du test N°2 (5,87) mais très nettement supérieur à la valeur obtenue dans le cadre du test N°1 (3,79).

### Observations

Une comparaison des valeurs obtenues dans le cadre des tests N°1, N°2 et N°3 mène au constat surprenant que le procédé selon la figure 2 (tableaux 1A à 1G) permet d'obtenir des seringues 1 dont les propriétés de glissement sont nettement améliorées et relativement durables dans le temps par le fait :
- d'inverser l'ordre des étapes de siliconage et de traitement corona décrit dans l'art antérieur constitué par le document US 2012/0277686 A1, et
- de ne prévoir qu'une unique étape de traitement corona,
- de soumettre ensuite le corps 2 de seringue à une irradiation par rayonnement gamma.

On constate également que l'ajout de l'étape c1) de traitement corona dans le procédé de la figure 3 (décrit dans l'art antérieur constitué par le document US 2008/071228 A1) améliore quelque peu les propriétés de glissement des seringues 1 par rapport à celles obtenues par un procédé utilisant l'ordre des étapes de siliconage et de traitement corona décrit dans l'art antérieur constitué par le document US 2012/0277686 A1. Toutefois, le procédé de la figure 2 mène à des propriétés de glissement encore meilleures, et présente l'avantage d'être moins long.

La présente invention n'est pas limitée aux modes de réalisation qui ont été explicitement décrits, mais elle en inclut les diverses variantes et généralisations contenues dans le domaine des revendications ci-après.

## Revendications

1. Procédé de préparation d'une seringue (1), comprenant les étapes successives suivantes :
a) fournir un corps (2) de seringue ayant une surface intérieure annulaire (2d), ledit corps (2) s'étendant autour d'un axe de symétrie longitudinal (1-1) entre une extrémité distale (2b) à orifice de sortie (2c) et une extrémité proximale (2a) par laquelle est destinée à être introduite une tête de piston (4) comprenant un joint (4a) destiné à venir en contact étanche contre la surface intérieure annulaire (2d),
b) appliquer un traitement corona à ladite surface intérieure annulaire (2d),
c) revêtir ladite surface intérieure annulaire (2d) d'une couche de silicone de façon à obtenir un corps (2) de seringue à surface intérieure annulaire (2d) revêtue de silicone,
d) irradier par rayonnement gamma le corps (2) de seringue
**caractérisé en ce qu'**il n'est appliqué qu'une unique étape de traitement corona.

2. Procédé selon la revendication 1, **caractérisé en ce que**, lors de l'étape d), le rayonnement gamma est appliqué selon une dose comprise entre 10 et 50 KGy.

3. Procédé selon l'une des revendications 1 ou 2, **caractérisé en ce que**, après l'étape b), la surface intérieure annulaire (2d) présente une tension de surface supérieure ou égale à 32 dyne/cm, et de préférence comprise entre 35 et 54 dyne/cm.

4. Procédé selon l'une quelconque des revendications 1 à 3, **caractérisé en ce que**, lors de l'étape b), le temps de traitement corona est compris entre 0,5 et 3 secondes, de préférence entre 0,75 et 1,5 secondes.

5. Procédé selon l'une quelconque des revendications 1 à 4, **caractérisé en ce que**, lors de l'étape b), le traitement corona est réalisé avec une intensité de courant électrique comprise entre 0,025 et 0,075 mA, de préférence entre 0,04 et 0,06 mA.

6. Procédé selon l'une quelconque des revendications 1 à 5, **caractérisé en ce que**, lors de l'étape c), on revêt la surface intérieure annulaire (2d) par projection de silicone au moyen d'une buse de pulvérisation (10) déplacée en translation depuis l'extrémité distale (2b) vers l'extrémité proximale (2a).

7. Procédé selon la revendication 6, **caractérisé en ce que**, lors de l'étape c), l'orifice de sortie (2c) est obstrué de façon étanche par un bouchon (3).

8. Procédé selon l'une des revendications 6 ou 7, **caractérisé en ce que**, lors de l'étape c), la buse de pulvérisation (10) est déplacée selon l'axe longitudinal (1-1) à une vitesse comprise entre 120 et 200 mm/s.

9. Procédé selon l'une quelconque des revendications 1 à 8, **caractérisé en ce que** le corps (2) de seringue a une surface intérieure annulaire (2d) en copolymère cyclo-oléfine (COC), en polymère cyclo-oléfine (COP), en verre, en polytétrafluoroéthylène (PTFE), en polychlorure de vinyle (PVC), en polypropylène (PP), en polyéthylène (PET), en polycarbonate (PC), ou en polystyrène (PS).

10. Procédé selon l'une quelconque des revendications 1 à 9, **caractérisé en ce qu'**on fournit un joint (4a) de tête de piston (4) de seringue en bromobutyle, en chlorobutyle, ou en bromochlorobutyle.

11. Procédé selon l'une quelconque des revendications 1 à 10, **caractérisé en ce qu'**on fournit un joint (4a) de tête de piston (4) de seringue présentant, avant introduction dans le corps (2) de seringue, un diamètre extérieur (D4) qui est supérieur de 0,27 mm à 0,47 mm au diamètre intérieur (D2d) de la surface intérieure annulaire (2d) avant son revêtement par le silicone.

12. Procédé selon l'une quelconque des revendications 1 à 11, **caractérisé en ce qu'**on fournit un joint (4a) de tête de piston (4) de seringue présentant une dureté comprise entre 50 et 56 Shore A.

13. Procédé selon l'une quelconque des revendications 1 à 12, **caractérisé en ce qu'**on fournit un joint (4a) de tête de piston (4) de seringue comportant un revêtement de silicone préalablement à son introduction dans le corps (2) de seringue.

14. Procédé selon l'une quelconque des revendications 1 à 13, **caractérisé en ce que** le corps (2) de seringue est rempli d'une substance liquide et assemblé avec une tête de piston (4) comprenant un joint (4a) destiné à venir en contact étanche contre la surface intérieure annulaire (2d) pour former une seringue (1), laquelle seringue (1) est de préférence soumise à une étape de stérilisation.

15. Ensemble pour la fabrication d'une seringue (1), ledit ensemble comportant une tête de piston (4) de seringue et un corps (2) de seringue ayant une surface intérieure annulaire (2d), **caractérisé en ce qu'**ont été successivement appliquées à la surface intérieure annulaire (2d) une unique étape de traitement corona suivie d'une étape de revêtement d'une couche de silicone, de préférence suivie d'une étape d'irradiation par rayonnement gamma.

16. Ensemble selon la revendication 15, **caractérisé en ce que**, après l'étape de traitement corona, la surface intérieure annulaire (2d) présente une tension de surface supérieure ou égale à 32 dyne/cm, et de préférence comprise entre 35 et 54 dyne/cm.

17. Ensemble selon l'une des revendications 15 ou 16, **caractérisé en ce que** la surface intérieure annulaire (2d) comporte en moyenne moins de 130 µg de silicone par cm², de préférence moins de 80 µg de silicone par cm².

18. Seringue (1) préparée par l'assemblage d'une tête de piston (4) de seringue et d'un corps (2) de seringue d'un ensemble selon l'une quelconque des revendications 15 à 17.

19. Seringue selon la revendication 18, **caractérisée en ce que** :
- la surface intérieure annulaire (2d) a un diamètre intérieur (D2d) de 5 mm,
- l'initiation du déplacement de la tête de piston (4) en direction de l'extrémité distale nécessite l'application d'un effort de poussée (Fs) inférieur à 7,5 N.

## Patentansprüche

1. Verfahren zur Herstellung einer Spritze (1), umfassend die folgenden aufeinanderfolgenden Schritte:
a) Bereitstellen eines Körpers (2) der Spritze mit einer ringförmigen Innenoberfläche (2d), wobei sich besagter Körper (2) um eine Längssymmetrieachse (I-I) zwischen einem distalen Ende (2b) mit Auslassöffnung (2c) und einem proximalen Ende (2a) erstreckt, das dazu vorgesehen ist einen Kolbenkopf (4) einzuführen, der eine Dichtung (4a) umfasst, die dazu vorgesehen ist, dichtend gegen die ringförmige Innenoberfläche (2d) in Kontakt zu kommen,
b) Anwenden einer Coronabehandlung auf besagte ringförmige Innenoberfläche (2d),
c) Beschichten besagter ringförmiger Innenoberfläche (2d) mit einer Silikonschicht, um so einen Körper (2) der Spritze mit einer ringförmigen Innenoberfläche (2d) zu erhalten, die mit Silikon beschichtet ist,
d) Bestrahlen des Körpers (2) der Spritze mit Gammastrahlung
**dadurch gekennzeichnet, dass** nur ein einziger Schritt Coronabehandlung angewendet wird.

2. Verfahren nach Anspruch 1, **dadurch gekennzeichnet, dass** beim Schritt d) die Gammastrahlung mit einer Dosis angewendet wird, die zwischen 10 und 50 KGy liegt.

3. Verfahren nach einem der Ansprüche 1 oder 2, **dadurch gekennzeichnet, dass** nach Schritt b) die ringförmige Innenoberfläche (2d) eine Oberflächenspannung aufweist, die größer oder gleich 32 Dyn/cm und vorzugsweise zwischen 35 und 54 Dyn/cm liegt.

4. Verfahren nach einem der Ansprüche 1 bis 3, **dadurch gekennzeichnet, dass** beim Schritt b) die Dauer der Coronabehandlung zwischen 0,5 und 3 Sekunden liegt, vorzugsweise zwischen 0,75 und 1,5 Sekunden.

5. Verfahren nach einem der Ansprüche 1 bis 4, **dadurch gekennzeichnet, dass** beim Schritt b) die Coronabehandlung mit einer elektrischen Stromstärke durchgeführt wird, die zwischen 0,025 und 0,075 mA liegt, vorzugsweise zwischen 0,04 und 0,06 mA.

6. Verfahren nach einem der Ansprüche 1 bis 5, **dadurch gekennzeichnet, dass** beim Schritt c) die ringförmige Innenoberfläche (2d) von Silikon mittels einer Pulversprühdüse (10) beschichtet wird, die translatorisch vom distalen Ende (2b) hin zum proximalen Ende (2a) bewegt wird.

7. Verfahren nach Anspruch 6, **dadurch gekennzeichnet, dass** beim Schritt c) die Auslassöffnung (2c) durch einen Stopfen (3) dichtend verschlossen wird.

8. Verfahren nach einem der Ansprüche 6 oder 7, **dadurch gekennzeichnet, dass** beim Schritt c) die Pulversprühdüse (10) entlang der Längsachse (I-I) mit einer Geschwindigkeit bewegt wird, die zwischen 120 und 200 mm/s liegt.

9. Verfahren nach einem der Ansprüche 1 bis 8, **dadurch gekennzeichnet, dass** der Körper (2) der Spritze eine ringförmige Innenoberfläche (2d) mit Cyclo-Olefin-Copolymer (COC), mit Cyclo-Olefin-Polymer-Olefin (COP), mit Glas, mit Polytetrafluorethylen (PTFE), mit Polyvinylchlorid (PVC), mit Polypropylen (PP), mit Polyethylen (PET), mit Polycarbonat (PC) oder mit Polystyrol (PS) hat.

10. Verfahren nach einem der Ansprüche 1 bis 9, **dadurch gekennzeichnet, dass** eine Dichtung (4a) des Kolbenkopfes (4) der Spritze mit Brombutyl, mit Chlorbutyl, oder mit Bromchlorbutyl bereitgestellt wird.

11. Verfahren nach einem der Ansprüche 1 bis 10, **dadurch gekennzeichnet, dass** eine Dichtung (4a) des Kolbenkopfes (4) der Spritze bereitgestellt wird, die vor dem Einführen in den Körper (2) der Spritze einen Außendurchmesser (D4) aufweist, der um 0,27 mm bis 0,47 mm größer ist als der Innendurchmesser (D2d) der ringförmigen Innenoberfläche (2d) vor ihrer Beschichtung mit dem Silikon.

12. Verfahren nach einem der Ansprüche 1 bis 11, **dadurch gekennzeichnet, dass** eine Dichtung (4a) des Kolbenkopfes (4) der Spritze bereitgestellt wird, die eine Härte aufweist, die zwischen 50 und 56 Shore A liegt.

13. Verfahren nach einem der Ansprüche 1 bis 12, **dadurch gekennzeichnet, dass** eine Dichtung (4a) des Kolbenkopfes (4) der Spritze bereitgestellt wird, die eine Silikonbeschichtung vor ihrem Einführen in den Körper (2) der Spritze aufweist.

14. Verfahren nach einem der Ansprüche 1 bis 13, **dadurch gekennzeichnet, dass** der Körper (2) der Spritze mit einer flüssigen Substanz gefüllt und mit einem Kolbenkopf (4) zusammengebaut ist, der eine Dichtung (4a) umfasst, die dazu vorgesehen ist, dichtend gegen die ringförmige Innenoberfläche (2d) in Kontakt zu kommen, um eine Spritze (1) zu bilden, wobei die Spritze (1) vorzugsweise einem Sterilisationsschritt unterzogen wird.

15. Baugruppe zur Herstellung einer Spritze (1), wobei besagte Baugruppe einen Kolbenkopf (4) einer Spritze und einen Körper (2) der Spritze aufweist, der eine ringförmige Innenoberfläche (2d) hat, **dadurch gekennzeichnet, dass** nacheinander auf die ringförmige Innenoberfläche (2d) ein einziger Schritt Coronabehandlung, gefolgt von einem Schritt des Beschichtens einer Silikonschicht, vorzugsweise gefolgt von einem Schritt der Bestrahlung mit Gammastrahlung, angewendet werden.

16. Baugruppe nach Anspruch 15, **dadurch gekennzeichnet, dass** nach dem Schritt Coronabehandlung die ringförmige Innenoberfläche (2d) eine Oberflächenspannung hat, die größer oder gleich 32 Dyn/cm und vorzugsweise zwischen 35 und 54 Dyn/cm liegt.

17. Baugruppe nach einem der Ansprüche 15 oder 16, **dadurch gekennzeichnet, dass** die ringförmige Innenoberfläche (2d) im Mittel weniger als 130 µg Silikon pro cm², vorzugsweise weniger als 80 µg Silikon pro cm² aufweist.

18. Spritze (1), hergestellt durch Zusammenbau eines Kolbenkopfes (4) einer Spritze und eines Körpers (2) der Spritze einer Baugruppe nach einem der Ansprüche 15 bis 17.

19. Spritze nach Anspruch 18, **dadurch gekennzeichnet, dass**:
- die ringförmige Innenoberfläche (2d) einen Innendurchmesser (D2d) von 5 mm hat,
- eine Initiierung der Bewegung des Kolbenkopfes (4) in Richtung des distalen Endes die Anwendung einer Schubkraft (Fs) von weniger als 7,5 N erfordert.

## Claims

1. Method for preparing a syringe (1), comprising the following successive steps:
a) providing a syringe body (2) which has an annular inner surface (2d), said body (2) extending about a longitudinal axis of symmetry (I-I) between a distal end (2b) with an outlet orifice (2c) and a proximal end (2a) via which a plunger head (4) is intended to be introduced, said plunger head comprising a seal (4a) intended to come into leaktight contact against the annular inner surface (2d),
b) applying a corona treatment to said annular inner surface (2d),
c) coating said annular inner surface (2d) with a layer of silicone so as to obtain a syringe body (2) with a silicone-coated annular inner surface (2d),
d) irradiating the syringe body (2) with gamma radiation,
**characterized in that** only one corona treatment step is applied.

2. Method according to Claim 1, **characterized in that**, during step d), the gamma radiation is applied at a dose of between 10 and 50 KGy.

3. Method according to either of Claims 1 and 2, **characterized in that**, after step b), the annular inner surface (2d) exhibits a surface tension of greater than or equal to 32 dyne/cm, and preferably of between 35 and 54 dyne/cm.

4. Method according to any one of Claims 1 to 3, **characterized in that**, during step b), the corona treatment time is between 0.5 and 3 seconds, preferably between 0.75 and 1.5 seconds.

5. Method according to any one of Claims 1 to 4, **characterized in that**, during step b), the corona treatment is carried out with an electrical current intensity of between 0.025 and 0.075 mA, preferably between 0.04 and 0.06 mA.

6. Method according to any one of Claims 1 to 5, **characterized in that**, during step c), the annular inner surface (2d) is coated by silicone spraying using a spray nozzle (10) moved translationally from the distal end (2b) to the proximal end (2a).

7. Method according to Claim 6, **characterized in that**, during step c), the outlet orifice (2c) is blocked in a leaktight manner by a stopper (3).

8. Method according to either of Claims 6 and 7, **characterized in that**, during step c), the spray nozzle (10) is moved along the longitudinal axis (I-I) at a speed of between 120 and 200 mm/s.

9. Method according to any one of Claims 1 to 8, **characterized in that** the syringe body (2) has an annular inner surface (2d) made of cyclo-olefin copolymer (COC), of cyclo-olefin polymer (COP), of glass, of polytetrafluoroethylene (PTFE), of polyvinyl chloride (PVC), of polypropylene (PP), of polyethylene (PET), of polycarbonate (PC) or of polystyrene (PS).

10. Method according to any one of Claims 1 to 9, **characterized in that** a syringe plunger head (4) seal (4a) made of bromobutyl, of chlorobutyl or of bromochlorobutyl is provided.

11. Method according to any one of Claims 1 to 10, **characterized in that** a seal (4a) of the syringe plunger head (4), having, before introduction into the syringe body (2), an outer diameter (D4) which is from 0.27 mm to 0.47 mm greater than the inner diameter (D2d) of the annular inner surface (2d) before coating thereof with silicone, is provided.

12. Method according to any one of Claims 1 to 11, **characterized in that** a syringe plunger head (4) seal (4a) having a hardness of between 50 and 56 Shore A is provided.

13. Method according to any one of Claims 1 to 12, **characterized in that** a syringe plunger head (4) seal (4a) comprising a silicone coating prior to the introduction thereof into the syringe body (2) is provided.

14. Method according to any one of Claims 1 to 13, **characterized in that** the syringe body (2) is filled with a liquid substance and assembled with a plunger head (4) comprising a seal (4a) intended to come into leaktight contact against the annular inner surface (2d) so as to form a syringe (1), which syringe (1) is preferably subjected to a sterilization step.

15. Set for the manufacture of a syringe (1), said set comprising a syringe plunger head (4) and a syringe body (2) which has an annular inner surface (2d), **characterized in that** a single corona treatment step, followed by a step of coating with a silicone layer, preferably followed by a step of irradiation with gamma radiation, have been successively applied to the annular inner surface (2d).

16. Set according to Claim 15, **characterized in that**, after the corona treatment step, the annular inner surface (2d) has a surface tension of greater than or equal to 32 dyne/cm, and preferably of between 35 and 54 dyne/cm.

17. Set according to either of Claims 15 and 16, **characterized in that** the annular inner surface (2d) comprises on average less than 130 µg of silicone per cm², preferably less than 80 µg of silicone per cm².

18. Syringe (1) prepared by assembling a syringe plunger head (4) and a syringe body (2) of a set according to any one of Claims 15 to 17.

19. Syringe according to Claim 18, **characterized in that**:
- the annular inner surface (2d) has an inner diameter (D2d) of 5 mm,
- the initiation of the movement of the plunger head (4) in the direction of the distal end requires the application of a thrusting force (Fs) of less than 7.5 N.
